# EUROPÄISCHE PATENTSCHRIFT

(11) **EP 1 629 831 B1**
(45) Veröffentlichungstag und Bekanntmachung des Hinweises auf die Patenterteilung: **09.01.2008**
(21) Anmeldenummer: 05012792.7
(22) Anmeldetag: 14.06.2005
(51) Int. Cl.: A61K 9/00, A61K 9/50, A61P 27/02

(54) **Verwendung eines Glaskörpertamponadepräparats**
Use of a vitreous humor tamponade
Utilisation d'une tamponade pour le corps vitreux

(30) Priorität: 20.08.2004 DE 102004040458
(43) Veröffentlichungstag der Anmeldung: 01.03.2006
(73) Patentinhaber: Meinert, Hasso, Professor Dr., 89231 Neu-Ulm (DE)
(72) Erfinder: Meinert, Hasso, Professor Dr., 89231 Neu-Ulm (DE)
(74) Vertreter: Eisenführ, Speiser & Partner

(56) Entgegenhaltungen:
- EP-A- 0 288 909
- WO-A-95/27482
- DE-A1- 4 220 882
- US-A- 4 180 467
- US-A- 4 844 905

## Beschreibung

Die Erfindung betrifft die Verwendung eines Glaskörpertamponadepräparats gemäß dem Oberbegriff des Patentanspruches 1.

Aus QE 42 20 882 A1 ist eine Glaskörpertamponadeflüssigkeit bekannt, welche in den Glaskörperraum eines nicht vitrektomierten Auges zu injizieren ist. Diese Flüssigkeit enthält wenigstens ein bei Normalbedingungen flüssiges Perfluorcarbon, das in Folge seines Eigendampfdruckes bei Körpertemperatur (etwa 37°C) nach der Injektion in den Glaskörperraum zur Verdrängung des Wasseranteils aus dem Glaskörperraum in die Gasphase übergeht. Im Perfluorcarbon kann ein Gas gelöst werden.

Aus Graefe's Arch Clin Exp Ophthalmol (1998) 236:709-712 ist die Verwendung von Perfluormethylcyclopentan (CF₃-cycloC₅F₉, FMCP) als Glaskörpertamponadeflüssigkeit zur Beseitigung des Wasseranteils aus dem Glaskörperraum infolge von Gasbildung in Tierversuchen beschrieben.

Bei der internen Tamponade (pneumatische Retinopexie) wird ein inertes expansibles Gas z. B. SF₆ in den mittleren Glaskörperraum möglichst unmittelbar vor die abgelöste Netzhaut injiziert. Hierbei kann aufgrund der resultierenden Augendruckerhöhung höchstens ein Volumen von 0,5 ml injiziert werden. Dieses Volumen vergrößert sich zwar durch Aufnahme von Stickstoff aus der umgebenden Flüssigkeit, jedoch ist hierbei eine vollständige Tamponade des gesamten Glaskörperraumes ohne Vitrektomie nicht möglich.

Aufgabe der Erfindung ist es, ein weiteres Glaskörpertamponadepräparat zu schaffen, mit dem ohne vorherige Glaskörperabsaugung ein zumindest passagerer und möglichst vollständiger Glaskörperersatz erreicht wird.

Diese Aufgabe wird durch die Merkmale des Patentanspruches 1 gelöst. Die Unteransprüche enthalten Weiterbildungen der Erfindung.

Das bei der Erfindung zur Anwendung kommende Glaskörpertamponadepräparat zeichnet sich dadurch aus, dass wenigstens ein als Feststoffpartikel vorliegender Reaktant zur Anwendung kommt, welcher im wässrigen Medium der Augenflüssigkeit unter Bildung eines Inertgases chemisch reagiert, wobei der Reaktant in einem biokompatiblen Material verkapselt ist, das im wässrigen Medium der Augenflüssigkeit retardierend löslich ist. Dieses Tamponademedium kann in einem einmaligen Schritt mittels einer herkömmlichen Kanüle in den Glaskörperraum injiziert werden und gewährleistet über einen längeren Zeitraum den erforderlichen Augendruck von maximal 30 mm Hg. Der Reaktant oder die Reaktanten des Tamponademediums, welches in Form von insbesondere Mikrokapseln mit einem an herkömmliche Kanülenlumen angepassten maximalen Durchmesser von 1,1 mm vorliegt, wird bzw. werden bei verzögerter chemischer Reaktion im wässrigen Medium der Augenflüssigkeit unter Volumen- und Druckzunahme umgesetzt. Hierbei ist über einen längeren Zeitraum hin der erforderliche Augendruck von maximal 30 mm Hg gewährleistet. Der maximale Durchmesser der Kapseln kann in Abhängigkeit von den verwendeten Kanülen auch kleiner oder größer sein. Die Kapseln können kugelförmig, ellipsoidförmig oder mit ähnlich abgerundeten Formen ausgebildet sein.

Als langsam im wässrigen Medium der Augenflüssigkeit lösliche und biokompatible Verkapselungsmaterialien können Gelatine, Kollagene, Albumine, Polysaccharide (Carboxymethylcellulose), Polyethylenglykoldimethylester, Polydextrane, Polylactide u.a. zum Einsatz kommen. Die Verkapselung, insbesondere Mikroverkapselung, erfolgt in an sich bekannter Weise. Dies geschieht beispielsweise dadurch, dass die Mikropartikel des Reaktanten im stöchiometrischen Mengenverhältniss gemäß der jeweiligen Reaktionsgleichung bei erhöhter Temperatur unter Ausschluss von Wasser gleichzeitig mit dem Verkapselungsmaterial versprüht und damit umhüllt werden. Ein anderes Umhüllungsverfahren besteht darin, dass die Reaktanten jeder für sich in das flüssige Verkapselungsmaterial eingebracht und das erhaltene Produkt danach im festen Zustand auf die erforderliche Größe vermahlen wird. Falls erforderlich, können dies Partikel einer nochmaligen Verkapselung unterzogen werden.

Für medizinische Zwecke müssen Reaktanten und Verkapselungsmaterialien biokompatibel sein. Die Dichte der Mikrokapseln kann vorzugsweise von 0,90 bis 1,15 g/cm³ betragen. Die Dichte des verkapselten Produktes lässt sich durch die Auswahl und das mengenmäßige Verhältnis von Verkapselungsmaterial zu Reaktanten einstellen. Je höher der Anteil am spezifisch leichteren Verkapselungsmaterial, umso niedriger ist die Dichte der Mikrokapseln.

Die zu verwendenden Umhüllungsmaterialien haben unterschiedliche Quell- und Löseeigenschaften im wässrigen Medium. Hierdurch lassen sich die nachfolgenden Prozesse bei der Gasbildung vorab zeitlich steuern. Da das Umhüllungsmaterial nur langsam im wässrigen Medium der Augenflüssigkeit als diffusionskontrollierter Prozess im Sinne von Retardkapseln aufgelöst wird, kann die chemische Reaktion des jeweiligen Reaktanten mit dem wässrigen Medium auch nur verzögert erfolgen.

Diese chemische Reaktion kann in Form einer Hydrolyse, doppelter Umsetzung oder Säure-Base-Reaktion ablaufen. Durch die dabei stattfindende Gasfreisetzung und Volumenzunahme erfolgt im geschlossenen System eine Druckzunahme und Wasserverdrängung und damit die Tamponadewirkung im Glaskörperraum des Auges.

Dieser Effekt erfolgt durch die oben geschilderten Vorgänge diffusionskontrolliert und somit verzögert.

Weiterhin bedingen die vorzugsweise vorgesehene Kugelform oder ähnlich abgerundete, z. B. ellipsoide Form der Mikrokapseln und das Einsetzen der Reaktion von der Oberfläche her, dass abhängig von der Oberflächengröße die freigesetzte Gasmenge zeitlich anfangs größer ist als gegen Ende der Applikation.

Im Folgenden werden Beispiele für Reaktanten und entsprechende Reaktionen unter Gasfreisetzung im Sinne der Erfindung angegeben.

### Beispiel 1

3 NaH₂PO₄ + 6 NaHCO₃ → 3 Na₃PO₄ + 6 H₂O + 6 CO₂

9,0 mg Salze in stöchiometrischem Mengenverhältnis ergeben ca. 1,4 ml CO₂ als inertes Tamponadegas.

### Beispiel 2

Ein weiteres Reaktionssystem als Ausführungsbeispiel der Erfindung besteht in der Verwendung der dem Beispiel 1 entsprechenden Kaliumsalze KH₂PO₄ und KHCO₃ mit entsprechender Freisetzung von CO₂.

### Beispiel 3

Bei einem weiteren Reaktionssystem als Ausführungsbeispiel der Erfindung erfolgt die Bildung von CO₂, H₂O und tertiärem Phosphat im Sinne von Beispiel 1 und Beispiel 2 bei den Umsetzungen der Hydrogenphosphate und Hydrogencarbonate oder Carbonate von Natrium oder Kalium, z. B. nach folgender Formel:

**Me₂HPO₄ + MeHCO₃ -----> Me₃PO₄ + H₂O + CO₂**

wobei Me = Na, K

### Beispiel 4

Bei einem weiteren Reaktionssystem als Ausführungsbeispiel der Erfindung erfolgt die Bildung von CO₂, H₂O und Salz, wenn mehrbasische aliphatische Carbonsäuren oder Hydroxycarbonsäuren mit den Hydrogencarbonaten oder Carbonaten von Natrium, Kalium oder Ammonium umgesetzt werden.

So reagiert z. B. Citronensäure mit Ammoniumcarbonat zu CO₂, H₂O und Ammoniumcitrat nach folgender Gleichung:

**2 C₃H₄(OH) (COOH)₃ + 3 (NH₄)₂CO₃ ----> 2 C₃H₄(OH) (COONH₄) ₃ + 3 H₂O + 3 CO₂**

Für die Glaskörpertamponade kommt die in Beispiel 1 beschriebene doppelte Umsetzung von Natriumdihydrogenphosphat mit Natriumhydrogencarbonat in Gegenwart von Wasser vorzugsweise zur Anwendung.

Alle in den Ausführungsbeispielen beschriebenen Komponenten sind biokompatibel. Dies gilt sowohl für die eingesetzten Reaktanten als auch für die zur Anwendung kommenden Verkapselungsmaterialien. Ebenfalls biokompatibel sind die Umsetzungsprodukte, die alle wasserlöslich sind und nach kurzer zeit aus dem Auge ausgeschwemmt werden.

Der Kohlendioxid-Druck kann bei den genannten Beispielen schon deshalb in Grenzen gehalten werden, weil CO₂ durch das HCO₃' / CO₃" - Gleichgewicht abgepuffert wird.

Zusammen mit den Reaktanten lassen sich auch Medikamente, wie z. B. zur Entzündungsprophylaxe, verkapseln, die nach dem Injizieren ebenfalls allmählich, im Sinne eines slow drug release, freigesetzt werden.

## Patentansprüche

1. Verwendung wenigstens eines als Feststoffpartikel vorliegenden Reaktanten, welcher im wässrigen Medium der Augenflüssigkeit unter Bildung eines Inertgases chemisch reagiert, wobei der wenigstens eine Reaktant mit einem im wässrigen Medium retardierend löslichen und biokompatiblen Material verkapselt ist, zur Herstellung eines biokompatiblen Glaskörpertamponadepräparats zur Injektion in den Glaskörperraum eines nicht vitrektomierten Auges, um den Wasseranteil aus dem Glaskörperraum zu verdrängen.

2. Verwendung eines Glaskörpertamponadepräparats nach Anspruch 1 **dadurch gekennzeichnet, dass** das Inertgas CO₂ ist.

3. Verwendung eines Glaskörpertamponadepräparats nach Anspruch 1 oder 2, **dadurch gekennzeichnet, dass** das Inertgas durch Hydrolyse, doppelte Umsetzung oder Säure-Base-Reaktion gebildet ist.

4. Verwendung eines Glaskörpertamponadepräparats nach einem der Ansprüche 1 bis 3, **dadurch gekennzeichnet, dass** die Reaktanten Natriumdihydrogenphosphat und Natriumhydrogencarbonat sind.

5. Verwendung eines Glaskörpertamponadepräparats nach einem der Ansprüche 1 bis 3, **dadurch gekennzeichnet, dass** die Reaktanten Kaliumdihydrogenphosphat und Kaliumhydrogencarbonat sind.

6. Verwendung eines Glaskörpertamponadepräparats nach einem der Ansprüche 1 bis 3, **dadurch gekennzeichnet, dass** die Reaktanten Hydrogenphosphate und Hydrogencarbonate oder Carbonate von Natrium oder Kalium sind.

7. Verwendung eines Glaskörpertamponadepräparast nach einem der Ansprüche 1 bis 3, **dadurch gekennzeichnet, dass** der Reaktant oder die Reaktanten mehrbasische aliphatische Carbonsäuren oder Hydroxycarbonsäuren und die Hydrogencarbonate oder Carbonate von Natrium, Kalium oder Ammonium sind.

8. Verwendung eines Glaskörpertamponadepräparats nach einem der Ansprüche 1 bis 7, **dadurch gekennzeichnet, dass** das biokompatible Verkapselungsmaterial wenigstens eine der Substanzen: Gelatine, Kollagene, Albumine, Polysaccharide (Carboxymethylcellulose), Polyethyleneglykoldimethylester, Polydextrane, Polylactide u.a. aufweist.

9. Verwendung eines Glaskörpertamponadepräparats nach einem der Ansprüche 1 bis 8, **dadurch gekennzeichnet, dass** die Kapseln, insbesondere Mikrokapseln kugelförmig oder ähnlich abgerundet ausgebildet sind.

10. Verwendung eines Glaskörpertamponadepräparats nach einem der Ansprüche 1 bis 9, **dadurch gekennzeichnet, dass** der maximale Durchmesser der Kapseln an den Lumendurchmesser der Injektionskanüle angepasst ist.

11. Verwendung eines Glaskörpertamponadepräparats nach einem der Ansprüche 1 bis 10, **dadurch gekennzeichnet, dass** die Kapseln einen Durchmesser von höchstens 1,1 mm aufweise.

12. Verwendung eines Glaskörpertamponadepräparats nach einem der Ansprüche 1 bis 11, **dadurch gekennzeichnet, dass** zwei oder mehrere Reaktanten in einer gemeinsamen Verkapselung vorgesehen sind.

13. Verwendung eines Glaskörpertamponadepräparats nach einem der Ansprüche 1 bis 12, **dadurch gekennzeichnet, dass** für die jeweiligen Reaktanten separate Verkapselungen vorgesehen sind,

14. Verwendung eines Glaskörpertamponadepräparats nach einem der Ansprüche 1 bis 13, **dadurch gekennzeichnet, dass** die Reaktanten im stöchiometrischen Mengenverhältnis der jeweiligen Reaktionsgleichung zur Bildung des Inertgas im Präparat vorliegen.

15. Verwendung eines Glaskörpertamponadepräparats nach einem der Ansprüche 1 bis 14, **dadurch gekennzeichnet, dass** die Dichte der Kapseln 0,9 bis 1,15 g/cm³ beträgt.

16. Verwendung eines Glaskörpertamponadepräparats nach einem der Ansprüche 1 bis 15, **dadurch gekennzeichnet, dass** sowohl der Reaktant oder die Reaktanten als auch die Reaktionsprodukte biokompatibel sind.

17. Verwendung eines Glaskörpertamponadepräparats nach einem der Ansprüche 1 bis 16, **dadurch gekennzeichnet, dass** mit dem wenigstens einen Reaktanten wenigstens ein Medikament verkapselt ist.

## Claims

1. Use of at least one reactant present in the form of solid particles, which reacts chemically in the aqueous medium of the vitreous humour to form an inert gas, wherein the at least one reactant is encapsulated in a biocompatible material, which is soluble in a retarding manner in the aqueous medium, in order to provide a biocompatible vitreous-tamponade preparation for injection into the vitreous chamber of a non-vitrectomised eye, in order to expel the water content from the vitreous chamber.

2. Use of a vitreous-tamponade preparation according to claim 1,
**characterised in that**
the inert gas is CO₂.

3. Use of a vitreous-tamponade preparation according to claim 1 or 2,
**characterised in that**
the inert gas is formed by hydrolysis, double decomposition or acid-base reaction.

4. Use of a vitreous-tamponade preparation according to any one of claims 1 to 3,
**characterised in that**
the reactants are sodium hydrogen phosphate and sodium hydrogen carbonate.

5. Use of a vitreous-tamponade preparation according to any one of claims 1 to 3,
**characterised in that**
the reactants are potassium hydrogen phosphate and potassium hydrogen carbonate.

6. Use of a vitreous-tamponade preparation according to any one of claims 1 to 3,
**characterised in that**
the reactants are hydrogen phosphates and hydrogen carbonates or carbonates of sodium or potassium.

7. Use of a vitreous-tamponade preparation according to any one of claims 1 to 3,
**characterised in that**
the reactant or reactants are multi-basic, aliphatic carboxylic acids or hydroxycarboxylic acids, and the hydrogen carbonates or carbonates are of sodium, potassium or ammonium.

8. Use of the vitreous-tamponade preparation according to any one of claims 1 to 7,
**characterised in that**
the biocompatible encapsulation material provides at least one of the substances: gelatines, collagens, albumins, polysaccharides (carboxymethyl cellulose), polyethylene glycoldimethyl esters, polydextrans and polylactides, inter alia.

9. Use of the vitreous-tamponade preparation according to any one of claims 1 to 8,
**characterised in that**
the capsules, in particular, microcapsules, are formed in a spherical or similarly rounded shape.

10. Use of a vitreous-tamponade preparation according to any one of claims 1 to 9,
**characterised in that**
the maximum diameter of the capsules is adapted to the diameter of the lumen of the injection cannula.

11. Use of a vitreous-tamponade preparation according to any one of claims 1 to 10,
**characterised in that**
the capsules provide a maximum diameter of 1.1 mm.

12. Use of a vitreous-tamponade preparation according to any one of claims 1 to 11,
**characterised in that**
two or more reactants are provided in a common encapsulation.

13. Use of a vitreous-tamponade preparation according to any one of claims 1 to 12,
**characterised in that**
separate encapsulations are provided for each of the reactants.

14. Use of a vitreous-tamponade preparation according to any one of claims 1 to 13,
**characterised in that**
the reactants are present in the stoichiometric ratio of the respective reaction equation for the formation of the inert gas in the preparation.

15. Use of a vitreous-tamponade preparation according to any one of claims 1 to 14,
**characterised in that**
the density of the capsules is 0.9 to 1.15 g/cm ³

16. Use of a vitreous-tamponade preparation according to any one of claims 1 to 15,
**characterised in that**
both the reactant or reactants and also the reaction products are biocompatible.

17. Use of a vitreous-tamponade preparation according to any one of claims 1 to 16,
**characterised in that**
at least one drug is encapsulated with the at least one reactant.

## Revendications

1. Utilisation d'au moins un réactif présent sous forme de particules solides, qui réagit chimiquement dans le milieu aqueux de l'humeur de l'oeil en formant un gaz inerte, où le au moins un réactif est encapsulé par un matériau biocompatible et soluble avec retardement dans le milieu aqueux, pour la fabrication d'une préparation de tamponade de corps vitré biocompatible pour l'injection dans l'espace du corps vitré d'un oeil non soumis à une vitrectomie, pour refouler la part d'eau de l'espace du corps vitré.

2. Utilisation d'une préparation de tamponade de corps vitré suivant la revendication 1, **caractérisée en ce que** le gaz inerte est du CO₂.

3. Utilisation d'une préparation de tamponade de corps vitré suivant la revendication 1 ou 2, **caractérisée en ce que** le gaz inerte est formé par hydrolyse, double conversion ou réaction acide - base.

4. Utilisation d'une préparation de tamponade de corps vitré suivant l'une des revendications 1 à 3, **caractérisée en ce que** les réactifs sont du dihydrogénophosphate de sodium et du hydrogénocarbonate de sodium.

5. Utilisation d'une préparation de tamponade de corps vitré suivant l'une des revendications 1 à 3, **caractérisée en ce que** les réactifs sont du dihydrogénophosphate de potassium et du hydrogénocarbonate de potassium.

6. Utilisation d'une préparation de tamponade de corps vitré suivant l'une des revendications 1 à 3, **caractérisée en ce que** les réactifs sont des hydrogénophosphates et des hydro-génocarbonates ou des carbonates de sodium ou de potassium.

7. Utilisation d'une préparation de tamponade de corps vitré suivant l'une des revendications 1 à 3, **caractérisée en ce que** le réactif ou les réactifs sont des acides carboxyliques aliphatiques polybasiques ou des acides hydroxy-carboxyliques et les hydrogénocarbonates ou carbonates de sodium, de potassium ou d'ammonium.

8. Utilisation d'une préparation de tamponade de corps vitré suivant l'une des revendications 1 à 7, **caractérisée en ce que** le matériau d'encapsulage biocompatible présente entre autres, au moins l'une des substances: gélatines, collagènes, albumines, polysaccharides (carboxyméthylcellulose), polyéthylène-glycoldiméthylester, polydextranes, polylactides.

9. Utilisation d'une préparation de tamponade de corps vitré suivant l'une des revendications 1 à 8, **caractérisée en ce que** les capsules, en particulier des micro-capsules, sont formées en forme de sphères ou arrondies de manière analogue.

10. Utilisation d'une préparation de tamponade de corps vitré suivant l'une des revendications 1 à 9, **caractérisée en ce que** le diamètre maximal des capsules est adapté au diamètre du lumen des canules d'injection.

11. Utilisation d'une préparation de tamponade de corps vitré suivant l'une des revendications 1 à 10, **caractérisée en ce que** les capsules présentent un diamètre maximal de 1,1 mm.

12. Utilisation d'une préparation de tamponade de corps vitré suivant l'une des revendications 1 à 11, **caractérisée en ce que** deux ou plusieurs réactifs sont prévus dans un encapsulage commun.

13. Utilisation d'une préparation de tamponade de corps vitré suivant l'une des revendications 1 à 12, **caractérisée en ce que** des encapsulages séparés sont prévus pour les réactifs respectifs.

14. Utilisation d'une préparation de tamponade de corps vitré suivant l'une des revendications 1 à 13, **caractérisée en ce que** les réactifs sont présents dans la préparation en proportion stoechiométrique de l'équation de réaction respective pour la formation du gaz inerte.

15. Utilisation d'une préparation de tamponade de corps vitré suivant l'une des revendications 1 à 14, **caractérisée en ce que** la masse spécifique des capsules est de 0,9 à 1,15 g/cm³.

16. Utilisation d'une préparation de tamponade de corps vitré suivant l'une des revendications 1 à 15, **caractérisée en ce qu'**aussi bien le ou les réactifs que les produits de réaction sont biocompatibles.

17. Utilisation d'une préparation de tamponade de corps vitré suivant l'une des revendications 1 à 16, **caractérisée en ce qu'**au moins un médicament est encapsulé avec le au moins un réactif.
